# FASCICULE DE BREVET EUROPEEN

(11) **EP 0 527 079 B1**
(45) Date de publication et mention de la délivrance du brevet: **23.10.1996**
(21) Numéro de dépôt: 92402201.5
(22) Date de dépôt: 31.07.1992
(51) Int. Cl.: C07D 401/12, A61K 31/47

(54) **N-(isoquinolein-5 YL) sulfonyl azacycloalcanes, leur procédé de préparation et les compositions pharmaceutiques les contenant**
N-(Isochinolein-5-yl)-Sulfonyl Azazykloalkane, Verfahren zu deren Herstellung und diese enthaltende pharmazeutische Zusammenstellungen
N-(isoquinolein-5-yl)-sulfonyl azacycloalkanes, their process of preparation and pharmaceutical compositions containing them

(30) Priorité: 31.07.1991 FR 9109720
(43) Date de publication de la demande: 10.02.1993
(73) Titulaire: ADIR ET COMPAGNIE, F-92415 Courbevoie Cédex (FR)
(72) Inventeur: Peglion, Jean-Louis, F-78110 Le Vesinet (FR); Vilaine, Jean-Paul, F-92290 Chatenay Malabry (FR); Villeneuve, Nicole, F-92500 Rueil Malmaison (FR); Janiak, Philip, F-92110 Clichy (FR)
(74) Mandataire: Reverbori, Marcelle

(56) Documents cités:
- EP-A- 0 187 371
- EP-A- 0 287 696

## Description

La présente invention a pour objet de nouveaux N-[(isoquinoléin-5 yl)sulfonyl]azacycloalcanes, leur procédé de préparation et les compositions pharmaceutiques qui les renferment.

Elle concerne plus particulièrement les composés de formule générale (I) : 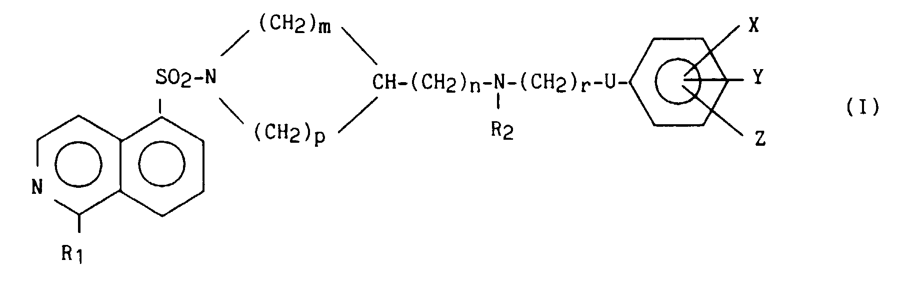 dans laquelle :
- n: est 0 ou un nombre entier de 1 à 3,
- m et p: sont des nombres entiers de 1 à 4 étant entendu que la somme m+p est 2, 3, 4 ou 5,
- r: est un nombre entier de 1 à 6,

R₁ représente un atome d'hydrogène, un groupement hydroxy ou un atome de chlore,
R₂ représente :
   - un atome d'hydrogène,
   - un groupement formyle,
   - un groupement -A, -CO-A, -CO-O-A, avec A signifiant un groupement alkyle inférieur, alkényle inférieur, alkynyle inférieur, cycloalkyle, ou cycloalkylalkyle inférieur,
   - un groupement -(CH₂)_{q}-phényl ou un groupement -(CH₂)_{q}-phényl substitué, avec q signifiant 0 ou un nombre entier de 1 à 4,
   - un groupement -CO-phényl ou un groupement -CO-phényl substitué,
   - un groupement -CO-O-phényl ou un groupement -CO-O-phényl substitué,
   - un groupement -CO-NR₄R₅
   où R₄, R₅ : - identiques ou différents, représentent un atome d'hydrogène ou un groupement choisi parmi alkyle inférieur, alkényl inférieur, alkynyl inférieur, phényle, phénylalkyle inférieur,
   - soit forment avec l'atome d'azote qui les porte un cycle saturé de 4 à 7 chaînons,
X, Y, Z identiques ou différents, représentent chacun indépendamment l'un de l'autre un atome d'hydrogène, un atome d'halogène, ou un groupement choisi parmi alkyle inférieur, alcoxy inférieur, nitro, amino, cyano, acétamido, carboxamido, ou X et Y, ou X et Z forment ensemble, avec les 2 atomes de carbone du noyau phényle qui les portent, un cycle furanique, dihydrofuranique, ou benzénique,
U représente un atome d'oxygène, un atome de soufre ou un groupement choisi parmi :
carbonyl, sulfinyl, sulfonyl, -NH-CO-, -CO-NH-, -O-(CH₂)_{r′}-O- avec r′ signifiant un nombre entier égal à 2 ou 3, -(CH₂)_{r˝}-O-(CH₂)_{r‴}-avec r˝ et r‴ signifiant des nombres entiers égaux à 1 ou 2, et 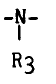
où
R₃ représente :
- un atome d'hydrogène,
- un groupement formyle,
- un groupement -A, -CO-A, ou -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué avec q signifiant 0 ou un nombre entier de 1 à 4,
- un groupement -CO-phényl ou un groupement -CO-phényl substitué,
- un groupement -CO-O-phényl ou un groupement -CO-O-phényl substitué,
- un groupement -CO-NR₄R₅ avec R₄ et R₅ tels que définis précédemment,

étant entendu que le terme "substitué" affectant les groupements -(CH₂)_{q}-phényl, -CO-phényl, ou -CO-O-phényl, signifie que ces groupements peuvent être substitués par un ou plusieurs radicaux choisis parmi : alkyle inférieur, alcoxy inférieur, hydroxy, atome d'halogène, et trifluorométhyle,
étant entendu que les termes "alkyle inférieur" et "alcoxy inférieur" signifient des groupements carbonés saturés linéaires ou ramifiés contenant de 1 à 6 atomes de carbone,
   que les termes "alkényle inférieur" et "alkynyle inférieur" désignent des groupements insaturés linéaires ou ramifiés contenant de 2 à 6 atomes de carbone,
   que le terme "cycloalkyl" désigne un cycle carboné saturé contenant de 3 à 8 chaînons.

On connaît de la littérature des composés de structure isoquinoléine sulfonamide montrant notamment une activité antiaggrégante (JP 63-325910), vasodilatatrice (EP 109023 et EP 287696) ou bronchorelaxante (USP 4857301).

L'état de l'art antérieur est également constitué des documents suivants : JP 02073067, EP 187371, EP 061673.

Des modifications importantes de structure ont conduit aux composés de la présente invention, lesquels présentent une activité pharmacologique anti-vasoconstrictrice particulièrement puissante et étendue, qu'on ne retrouve pas pour les composés de l'art antérieur.
La présente invention a également pour objet le procédé de préparation des composés de formule générale (I) caractérisé en ce que :
- on fait réagir un composé de formule (II) : 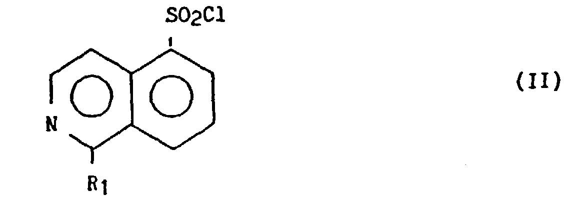 dans laquelle R₁ a la même signifcation que dans la formule générale (I),
- soit avec un composé de formule (III) : 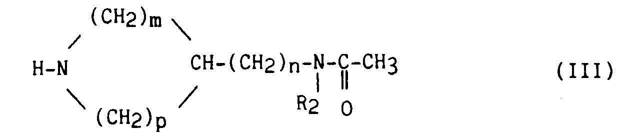 dans laquelle R₂, n, m et p ont la même signification que dans la formule générale (I),
   pour obtenir, après hydrolyse acide, un composé de formule (IV) : 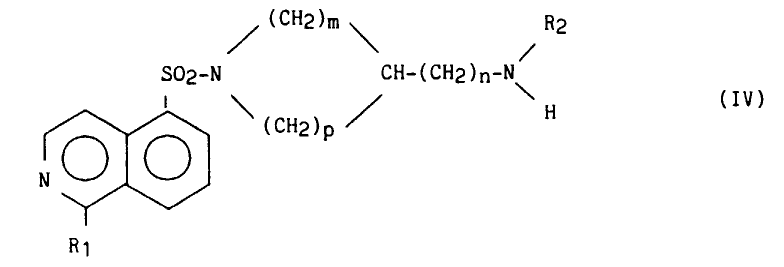 dans laquelle R₁, R₂, n, m et p sont tels que définis précédemment, que l'on fait réagir avec un composé de formule (V) : 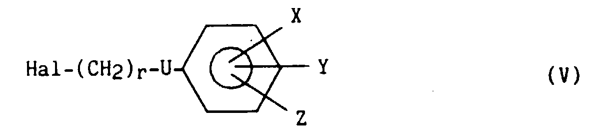 dans laquelle X, Y, Z, U et r ont la même signification que dans la formule générale (I) et Hal représente un atome d'halogène, pour obtenir un composé de formule (I) : 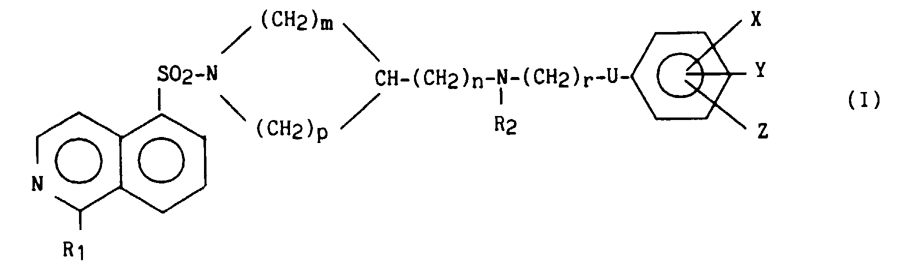 dans laquelle R₁, R₂, U, X, Y, Z, n, m, p et r sont tels que définis dans la formule générale (I),
- soit avec un composé de formule (VI) : 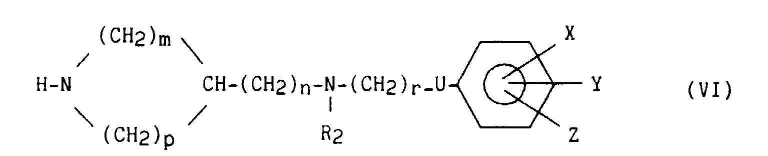 dans laquelle R₂, U, X, Y, Z, n, m, p et r sont tels que définis précédemment, pour obtenir un composé de formule (I), dont on sépare, le cas échéant, les isomères, et que l'on salifie, si on le désire, avec un acide pharmaceutiquement acceptable,
composé de formule (I) que l'on purifie, si on le désire, par une technique de cristallisation et/ou de chromatographie.

Les matières premières utilisées dans les procédés précédemment décrits sont soit des produits connus, soit des produits préparés à partir de substances connues, selon des procédés décrits pour préparer des produits analogues comme indiqués dans les exemples suivants.

Les composés de formule générale (I) peuvent être transformés en sels d'addition avec les acides, sels qui font à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthanesulfonique, iséthionique, benzènesulfonique,...

D'autre part, dans le cas où il existe un ou plusieurs carbones asymétriques, les composés de formule (I) peuvent se présenter sous forme de diastéréoisomères ou d'énantiomères, qui font à ce titre, sous forme pure ou sous forme de mélange, partie de l'invention.

Les composés de formule générale (I) et leurs sels d'addition pharmaceutiquement acceptables possèdent des propriétés thérapeutiques intéressantes, notamment dans le domaine cardio-vasculaire.

Les essais pharmacologiques réalisés in vitro ont montré que ces produits ont une puissante activité anti-vasoconstrictrice vis à vis des différents types de processus impliqués dans la contraction du muscle lisse et notamment vis à vis des processus intracellulaires mettant en jeu le calcium intracellulaire.

Ces propriétés permettent donc l'utilisation des composés de la présente invention comme médicament, et notamment dans le domaine cardiovasculaire dans le traitement et la prévention de l'ischémie myocardique et de ses différentes expressions cliniques comme l'angine de poitrine et l'infarctus du myocarde, mais également dans la prévention et le traitement des troubles du rythme cardiaque, du spasme vasculaire, de l'hypertension artérielle, des maladies vasculaires et de l'insuffisance cardiaque, et plus généralement dans le traitement et la prévention des troubles liés au vieillissement artériel et à l'athérosclérose.

Ces dérivés peuvent aussi être administrés pour la prévention des resténoses ou thromboses vasculaires après pontage, dilatation vasculaire notamment coronaire, ou autres formes de reperméabilisation vasculaire. Ils peuvent être également utilisés dans les pathologies métaboliques constituant un facteur de risque cardio-vasculaire tels que l'obésité, le diabète et les dyslipidémies.

En outre, l'effet régulateur du 'calcium intracellulaire de ces produits leur confère une possible utilisation thérapeutique à titre d'agents antiaggrégants plaquettaires et antithrombotiques ou d'agents relaxants des différents types de muscles lisses (autres que vasculaires déjà cités) : bronchiques, digestifs, urinaires, ou utérins.

De plus de nombreuses situations de souffrance tissulaire , qu'elles soient liées au vieillissement, à l'ischémie, à l'inflammation ou à une prolifération cellulaire, peuvent être traitées ou prévenues par les produits de la présente invention.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par la voie orale, rectale, intramusculaire, ou parentérale.

La posologie peut varier notablement selon l'âge et le poids du patient, la voie d'administration, la nature de la maladie et les traitements associés et consiste en prises de 1 à 100 mg, une à plusieurs fois par jour.

Les exemples suivants illustrent l'invention mais ne la limitent en aucune façon.

Les points de fusion sont déterminés à la platine chauffante de Köfler. Les spectres de résonnance magnétique nucléaire 1H (RMN) ont été réalisés en utilisant le tétraméthylsilane (TMS) comme référence interne. Les déplacements chimiques sont exprimés en partie par million (ppm). Les spectres infrarouge ont été effectués sous forme de pastille de bromure de potassium renfermant environ 1 % du produit à analyser.

### Exemple 1

### Fumarate de 1-[(isoquinoléin-5 yl)sulfonyl] 4-{N-(méthyl) N-[4-(p-fluorophénoxy)butyl]amino}pipéridine

### Stade A

### 1-(benzyl) 4-(méthylamino)pipéridine

On refroidit à 18 °C une solution de 1 mole de N-benzylpipérid-4-one dans 2 200 cm³ d'isopropanol. On ajoute à cette solution une solution fraîchement préparée de 3 moles de méthylamine dans 300 cm³ de méthanol. On laisse le mélange 1 heure à cette température.
On ajoute 1,66 mole d'hydroxyde de sodium en pastilles. On ajoute, à une température constante de 18 °C, 1,37 mole de borohydrure de sodium. On laisse revenir à température ambiante puis on laisse en contact 12 heures. On évapore les solvants, on reprend à l'éther puis on lave à l'eau. Après séchage et évaporation, on obtient une huile qui est le composé attendu. Rendement : 78 %.

### Stade B

### 1-(benzyl) 4-[N-(méthyl) N-(acétyl)amino]pipéridine

On prépare une solution formée de 132 g (0,647 mole) du composé obtenu au stade précédent et de 65,4 g de triéthylamine dans 1 300 cm³ de chlorure de méthylène. On ajoute lentement 45,9 cm³ de chlorure d'acétyle. On laisse en contact 2 heures. On évapore, on reprend à l'éther puis on lave à l'eau. Après séchage et évaporation du solvant, on obtient 128 g d'une huile qui correspond au composé désiré et qui sera utilisée sans autre purification.

### Stade C

### 4-[N-(méthyl) N-(acétyl)amino]pipéridine

On hydrogène 0,38 mole du composé obtenu au stade précédent dans 700 cm³ d'éthanol et 0,38 mole d'acide acétique sous une pression de 5 kg/cm² en présence de 9 g d'hydroxyde de palladium.
Après filtration et évaporation, on reprend le résidu par 500 cm³ de chlorure de méthylène, on basifie à froid, sous agitation, par 0,38 mole d'hydroxyde de sodium à 20 %. On décante, on sèche, puis on évapore.
On obtient 49,7 g du composé attendu.
Rendement 83 %.
Point du fusion : < 50 °C.

### Stade D

### 1-[(isoquinoléin-5 yl)sulfonyl] 4-[N-(méthyl) N-(acétyl)amino]pipéridine

On prépare une solution de 13,7 g (0,0879 mole) du composé obtenu au stade précédent, et de 22,5 g de diisopropyléthylamine dans 950 cm³ de chlorure de méthylène. On ajoute, sous agitation et à température ambiante, 23,3 g (0,0879 mole) de chlorhydrate du sulfochlorure de 5-isoquinoléine finement pulvérisé. On transvase en ampoule, on lave par 90 cm³ d'hydroxyde de sodium, puis à l'eau. On sèche et on évapore. On chromatographie l'huile obtenue sur silice avec comme système d'élution un mélange chlorure de méthylène/méthanol (95/5) pour obtenir 17 g d'un solide qui correspond au composé attendu.
Rendement : 58 %
Point de fusion : 194-196 °C.

### Stade E

### 1-[(isoquinoléin-5 yl)sulfonyl] 4-(méthylamino)pipéridine

On porte à reflux durant 12 heures un mélange composé de 0,051 mole du composé obtenu au stade précédent, de 160 cm³ d'acide chlorhydrique concentré et de 160 cm³ d'eau. On refroidit, on basifie par de l'hydroxyde de sodium à 20 %, puis on extrait à l'acétate d'éthyle. Après évaporation, on soumet l'huile obtenue à une chromatographie sur silice en utilisant comme système d'élution un mélange chlorure de méthylène/méthanol (80/20).
On obtient 4,5 g du composé désiré.

### Stade F

### 1-bromo 4-(p-fluorophénoxy)butane

On dissout 0,5 mole d'hydroxyde de potassium dans 200 cm³ de méthanol. On ajoute 0,5 mole de p-fluorophénol, 3,4 moles de 1,4-dibromobutane, et 3 mmoles d'iodure de potassium. On porte le mélange au reflux, sous agitation, pendant 24 heures.
Après évaporation du solvant, on reprend le résidu à l'éther et on effectue un lavage à l'eau puis à l'hydroxyde de sodium 1N. Après avoir éliminé l'excès de composé dibromé, on distille le résidu grâce à un système Kughelrohr.
On obtient une huile qui est le composé attendu.
Rendement : 66 %
Eb_{(1,33 Pa)} = 100 °C.

### Stade G

### 1-[(isoquinoléin-5 yl)sulfonyl] 4-{N-(méthyl) N-[4-(p-fluorophénoxy) butyl]amino}pipéridine

On prépare une solution formée de 5,4 g du composé obtenu au stade E, de 0,0177 mole du composé obtenu au stade F, 2,5 g de carbonate de potassium, et de 50 cm³ d'acétone.
On porte le mélange au reflux, sous agitation, pendant 12 heures. Après évaporation, on reprend à l'éther, puis on épuise la phase éthérée avec de l'acide chlorhydrique 1N. On basifie à froid les phases acides, on extrait à l'éther et on sèche. Après évaporation, on obtient 8,4 g d'une huile que l'on chromatographie sur silice avec pour système d'élution un mélange toluène/méthanol (95/5).
On obtient 3 g d'huile qui correspond au composé attendu.

### Stade H

### Le composé du titre

Reprendre les 3 grammes du composé du stade précédent par 18,5 cm³ d'une solution 0,172 molaire d'acide fumarique dans l'éthanol. On évapore à sec la solution obtenue, puis on reprend, sous agitation pendant 12 heures, par 10 cm³ d'acétate d'éthyle.
On obtient, sous forme de précipité, 1,1 g du composé attendu.
Point de fusion : 95 °C
Solvant de cristallisation : acétate d'éthyle.

### Exemple 2

### Dichlorhydrate de 1-[(isoquinoléin-5 yl)sulfonyl] 4-{N-(méthyl) N-[4-(oxo) 4-(p-fluorophényl)butyl]amino}pipéridine

### Stade A

### 1-(iode) 3-[(p-fluorophényl)carbonyl]propane

On porte à reflux pendant 24 heures, 20 g de 1-(chloro) 3-[(p-fluorophényl)carbonyl]propane dissous dans 100 cm³ de méthyléthylcétone, en présence de 18,5 g d'iodure de sodium. On isole après traitement 28 g du composé attendu.

### Stade B

### 1-[isoquinoléin-5 yl)sulfonyl] 4-{N-(méthyl) N-[4-(oxo) 4-(p-fluorophényl)butyL]amino}pipéridine.

On porte à reflux durant 12 heures, sous agitation, un mélange composé de 4,3 g du composé obtenu au stade A, de 4,5 g du composé obtenu au stade E de l'exemple 1, de 2 g de carbonate de potassium, et de 50 cm³ d'acétone. On procède ensuite de façon analogue au stade G de l'exemple 1 pour obtenir 2,1 g du composé attendu.

### Stade C

### Le composé du titre

On transforme, 2,1 g du composé obtenu au stade précédent en solution dans l'acétonitrile, en dichlorhydrate, par ajout de 3,4 cm³ d'éther chlorhydrique 3N. On obtient 2 g du produit désiré.
Point de fusion : 224-226 °C.
Solvant de cristallisation : acétonitrile.

### Caractéristiques spectrales

### Infrarouge :

- ν (NH⁺) :: 2800-1900 cm⁻¹
- ν (C = O) :: 1680 cm⁻¹
- ν (N-SO₂) :: 1163 cm⁻¹

### RMN :

- 1H :: 9,8 ppm (singulet) ;
- 2H+2H :: 8,8 à 8,5 ppm (singulet) ;
- 2H + 1 H :: 8 ppm (multiplet+triplet) ;
- 2H :: 7,35 ppm (triplet);
- 2H :: 4 ppm (doublet large) ;
- 2H+3H :: 3,5 à 2,9 ppm (2 multiplets) ;
- 3H+2H :: 2,65 ppm (singulet+ triplet) ;
- 2H+2H :: 2,3 à 1,9 ppm (2 multiplets) ;
- 2H :: 1,8 ppm (multiplet) ;
- 2H :: 11 ppm échangeables pour D20.

### Exemple 3

### Fumarate de 1-[(isoquinoléin-5 yl)sulfonyl] 4-{[N-(méthyl) N-[3-(p-fluorophénoxy)propyl]amino}méthyl}pipéridine

### Stade A

### [1-(phénylcarbonyl)pipérid-4 yl]carboxylate d'éthyle

On coule lentement 70 g (0,5 mole) du chlorure de l'acide benzoïque sur un mélange composé de 78,5 g d'isonipécotate d'éthyle, de 50,5 g de triéthylamine, et de 700 cm³ de chlorure de méthylène.
On laisse une nuit à température ambiante puis on évapore. On reprend à l'éther, on lave à l'eau et on sèche.
On obtient 106,8 g d'une huile qui correspond au composé attendu.

### Stade B

### Acide [1-(phénylcarbonyl)pipérid-4 yl]carboxylique

43,3 g (0,15 mole) du composé obtenu au stade précédent sont saponifiés par 16 cm³ d'hydroxyde de sodium 1N dans 160 cm³ d'éthanol à température ambiante. Après traitement, on obtient 31,8 g du composé attendu.
Rendement : 91 %
Point de fusion : 130-133 °C.

### Stade C

### N-méthyl [1-(phénylcarbonyl)pipérid-4 yl]carboxamide

On dissout 0,136 mole du composé obtenu au stade précédent dans 150 cm³ de chlorure de méthylène. On ajoute en une seule fois 22,1 g de carbonyldiimidazole. A la fin du dégagement gazeux, on ajoute par un goutte à goutte rapide une solution fraîchement préparée de 0,5 mole de méthylamine dans 150 cm³ de chlorure de méthylène.
On laisse 12 h sous agitation. On transvase en ampoule, on sèche à l'eau et on évapore.
Rendement : 58 %.
Point de fusion : 182-184 °C.

### Stade D

### 1-(benzyl) 4-[(méthylamino)méthyl] pipéridine.

On réduit 0,088 mole du composé obtenu au stade précédent dans 300 cm³ de tétrahydrofurane par addition d'une suspension de 6,7 g d'hydrure de lithium et d'aluminium dans 100 cm³ de tétrahydrofurane. On porte le mélange à reflux pendant 4 heures. Après traitement, on obtient 15,1 g d'huile qui correspond au composé attendu. Ce composé est purifié par chromatographie sur silice avec un mélange chlorure de méthylène/méthanol/hydroxyde d'ammonium (89/10/1) comme éluant.
Rendement : 52 %.

### Stade E

### 1-bromo 3-(p-fluorophénoxy)propane

En procédant comme dans le stade F de l'exemple 1, mais en remplaçant le 1,4-dibromobutane par le 1,3-dibromopropane, on obtient le composé attendu.

### Stade F

### 1-(benzyl) 4-{{N-(méthyl) N'-[3-(p-fluorophénoxy)propyl]amino}méthyl} pipéridine

On prépare un mélange composé de 8,9 g du composé obtenu au stade D, de 10,3 g du composé obtenu au stade E, de 6,4 g de carbonate de potassium et de 170 cm³ d'acétone. On porte le mélange au reflux pendant 12 heures. On concentre, on reprend à l'éther, on lave à l'eau, et on épuise la phase éthérée par de l'acide chlorhydrique 1N. On basifie à froid les phases acides, on extrait à l'éther. On sèche et on évapore pour conduire à 13,3 g d'une huile qui correspond au composé attendu et qui sera utilisée sans autre purification.
Rendement : 78 %.

### Stade G

### 4-{{N-(méthyl) N-3-(p-fluorophénoxy)propyl]amino}méthyl}pipéridine.

On hydrogène 0,036 mole du composé obtenu au stade précédent sous 5 kg de pression/cm² dans 150 cm³ d'éthanol et 2,1 g d'acide acétique, en présence de 1,4 g d'hydroxyde de palladium. Après traitement, on obtient 4,8 g du composé désiré
Rendement : 42 %.

### Stade H

### 1-[(isoquinoléin-5 yl)sulfonyl] 4-{{N-(méthyl) N-[3-(p-fluorophénoxy) propyl]amino}méthyl}pipéridine.

En procédant comme dans le stade D de l'exemple 1, mais en utilisant le composé obtenu au stade G du présent exemple, on obtient le composé désiré.

### Stade I

### Le composé du titre

On traite 3,6 g du composé obtenu au stade précédent par 40,7 cm³ d'une solution d'acide fumarique 0,172 molaire dans l'éthanol.
On obtient 2 g du composé du titre.
Point de fusion : 127-129 °C.
Solvant de cristallisation : éthanol.

### Exemple 4

### Fumarate de 1-[(isoquinoléin-5 yl)sulfonyl] 4-{N-(méthyl) N-[5-(p-fluorophénoxy)pentyl]amino}pipéridine

En procédant comme dans l'exemple 1, mais en remplaçant au stade F le 1,4-dibromobutane par le 1,5-dibromopentane, on obtient le composé du titre.
Rendement final : 16 %
Point de fusion : 150 °C
Solvant de cristallisation : acétate d'éthyle.

### Exemple 5

### Dichlorhydrate de 1-[(isoquinoléin-5-yl)sulfonyl] 4-[N-méthyl N-[4-(p-fluorophényl) 4-oxo butyl]amino méthyl]pipéridine

### Stade A

### 1-benzyl 4-{N-méthyl N-[4-(p-fluorophényl) 4-oxo-butyl]amino méthyl} pipéridine

En procédant comme au stade F de l'exemple 3, à partir de 1-benzyl 4-[méthylamino-méthyl]pipéridine et de 1-iodo 3-[(p-fluorophényl)carbonyl]propane, en présence de carbonate de potassium et dans l'acétone comme solvant, on obtient le composé attendu sous forme d'huile, avec un rendement de 77 %.

### Stade B

### 4-{N-méthyl N-[4-(p-fluorophényl) 4-oxo-butyl]amino méthyl} pipéridine

En procédant comme au stade G de l'exemple 3, on obtient, par hydrogénation du composé obtenu au stade A ci-dessus, le composé attendu, sous forme d'huile.

### Stade C

### 1-[(isoquinoléin-5 yl) sulfonyl] 4-{N-méthyl N-[4-(p-fluorophényl) 4-oxo butyl]amino méthyl} pipéridine

En procédant comme décrit au stade G de l'exemple 1, à partir du composé obtenu au stade B ci-dessus et du chlorure d'(isoquinoléin-5-yl)sulfonyle, on obtient le composé attendu sous forme d'huile.
Rendement : 29 %.

### Stade D

### Dichlorhydrate de 1-[(isoquinoiéin-5-yl)sulfonyl] 4-{N-méthyl N-[(4-(p-fluorophényl) 4-oxo-butyl]amino méthyl}pipéridine

En opérant comme au stade C de l'exemple 2, on transforme le composé obtenu au stade précédent en dichlorhydrate correspondant, PF (K) instantané : 148 °C.

### Exemple 6

### ETUDE PHARMACOLOGIQUE IN VITRO DES COMPOSÉS DE L'INVENTION

### A/ activité anti-vasoconstrictrice

### Matériel et méthodes

Les études sont réalisées sur des anneaux de 3 mm de longueur
- d'aortes prélevées chez des rats WISTAR (325-375 g) anesthésiés au pentobarbital sodique (30 mg/kg en intrapéritonéal)
- ou d'aortes prélevées chez des lapins de Nouvelle-Zélande (1,8-2 kg) également anesthésiés au pentobarbital sodique (30 mg/kg en intrapéritonéal).

Les anneaux vasculaires sont immergés dans une solution physiologique, normale ou dépourvue de calcium (+ 0,2mM du chélateur EGTA), thermostatée à 37 °C, aérée par un mélange 95 % O₂ + 5 % CO₂. Les anneaux sont reliés à un capteur de tension STATHAM (UC2-GOULD). Les tensions optimales pour chaque vaisseau sont appliquées et une période de stabilisation de 90 mn des préparations est respectée.

### a) Activité anti-vasoconstrictrice des composés de l'invention en milieu physiologique.

### Protocoles d'étude

- L'activité relaxante des composés de l'invention, testés en concentrations cumulatives additionnées toutes les 15 minutes, vis à vis de la contraction d'anneaux vasculaires induite par un milieu hyperpotassique (80 mM KCl, 37 mM NaCl), a permis le calcul de l'IC₅₀ (Concentration molaire qui inhibe de 50 % la contraction maximale).

### Résultats

Le tableau (T1) regroupe les IC50 obtenus avec les composés exemplifiés représentatifs des composés de l'invention vis à vis de la contraction d'aorte de rat induite par un milieu hyperpotassique.

**TABLEAU (T1)**

| **CONCENTRATION INHIBANT DE 50 % (IC**_{**50**}**) LA CONTRACTION D'AORTES DE RAT INDUITE PAR DEPOLARISATION POTASSIQUE** | | | |
|---|---|---|---|
| **N° de l'exemple testé** | 1 | 2 | 3 |
| **IC**_{**50**} **(en M)** | 9,2.10⁻⁶ | 3,3.10⁻⁶ | 1,5.10⁻⁶ |

### b) Activité anti-vasoconstrictrice des composés de l'invention en milieu sans calcium.

### Protocoles d'étude

L'effet inhibiteur d'une concentration des composés de l'invention incubée pendant 30 minutes, vis à vis d'une contraction vasculaire induite par la noradrénaline (10⁻⁶ M), est testé.

### Résultats

Le tableau (T2) montre l'activité des composés de l'invention vis à vis de la contraction d'aortes de lapin induite par la noradrénaline en milieu dépourvu de calcium.

**(T2)**

| **% D'INHIBITION DE LA CONTRACTION INDUITE PAR LA NORADRENALINE EN MILIEU SANS CALCIUM** | | |
|---|---|---|
| **N° de l'exemple testé** | **Concentration du composé** | |
| | **10**^{**-6**} **M** | **10**^{**-7**} **M** |
| 1 | 62 | |
| 2 | 86 | 50 |
| 3 | 82 | 43 |

### CONCLUSION

Les dérivés de l'invention exercent une activité inhibitrice remarquable vis à vis de la contraction vasculaire induite par une dépolarisation potassique. De façon très intéressante ces produits sont également actifs vis à vis d'une contraction induite par un médiateur vasoconstricteur, telle la noradrénaline, et ceci même en l'absence de calcium extracellulaire.

### B/ Activité protectrice sur le myocarde

### a) Effet des composés de l'invention sur une intoxication à la ouabaïne de l'oreillette gauche de cobaye

### Protocole d'étude

On prélève les oreillettes gauches sur des cobayes (350-450 g) anesthésiés au pentobarbital sodique (30 mg/kg). Les oreillettes sont accrochées à un capteur de tension STATHAM (UC2-GOULD) et la tension initiale appliquée est de 0,5 grammes.
On stimule les oreillettes électriquement à 1 Hz par l'intermédiaire d'électrodes de platine.
L'intoxication est réalisée par une additon de ouabaïne (10⁻⁶ M).
On ajoute les composés à tester 15 minutes avant l'addition de l'agent toxique.

### Résultats

| **Effet des produits des exemples 2 et 3 sur une intoxication à la ouabaïne de l'oreillette gauche de cobaye** | | | |
|---|---|---|---|
| | **SOLVANT H**_{**2**}**O (n=5)** | **Exemple 2 10-5 M (n=5)** | **Exemple 3 10-5 M (n=4)** |
| % CONTRACTION | | | |
| 10 MIN | 207,8 ± 17,4 | 184,0 ± 17,3 | 183,3 ± 20,7 |
| 30 MIN | 80,4 ± 19,4 | 138,8 ± 34,9 | 155,4 ± 15,0 |
| 60 MIN | 19,6 ± 7,7 | 112,2 ± 31,0 | 130,7 ± 34,5 |
| % CONTRACTURE | | | |
| 15 MIN | 2,4 ± 1,2 | 1,4 ± 1,4 | 0,9 ± 0,9 |
| 30 MIN | 40,3 ± 16,3 | 10,5 ± 4,7 | 7,7 ± 1,5 |
| 60 MIN | 93,2 ± 23,9 | 33,5 ± 16,4 | 21,8 ± 6,2 |

| **Effet des produits des exemples 1, 4 et 5 sur une intoxication à la ouabaïne de l'oreillette gauche de cobaye** | | | | | |
|---|---|---|---|---|---|
| | **SOLVANT DMSO** | **Exemple 1 10**^{**-5**} **M** | **Exemple 4 10**^{**-5**} **M** | **SOLVANT H**_{**2**}**O** | **Exemple 5 10-5 M** |
| % CONTRACTION | | | | | |
| 10 MIN | 202,6 ± 16,5 | 254,7 ± 22 | 213,9 ± 22 | 190,7 ± 14,8 | 206,1 ± 21 |
| 30 MIN | 69,0 ± 13,1 | 260,6 ± 29 | 208,6 ± 22 | 51,3 ± 6,6 | 186,1 ± 21,2 |
| 60 MIN | 24,7 ± 6,1 | 253,6 ± 26 | 211,1 ± 30 | 48,7 ± 15,0 | 141,3 ± 25,3 |
| % CONTRACTURE | | | | | |
| 15 MIN | 0 | 0 | 0 | 7,3 ± 3,4 | 0 |
| 30 MIN | 23,2 ± 8,4 | 0 | 0 | 70,2 ± 14,5 | 2,8 ± 2,1 |
| 60 MIN | 102,8 ± 12,8 | 4 ± 4 | 0 | 87,6 ± 9,8 | 16,5 ± 9,5 |

Ces résultats montrent que les composés de la présente invention s'opposent de façon importante au développement de la contracture (exprimée en % de la contraction developpée initiale) et à l'effondrement de la contraction développée liés aux effets toxiques de la ouabaïne.

### b) Effet protecteur des composés de l'invention sur coeur isolé soumis à une hypoxie-réoxygénation

### Protocole d'étude

On prélève le coeur sur des rats WISTAR (325-375 g). Le coeur est rapidement perfusé selon la technique de LANGENDORFF à une pression constante de 76 mmHg et stimulé électriquement à 5 Hz.
Le coeur est soumis à une hypoxie de 60 mn réalisée par l'administration d'un mélange gazeux 95 % N₂ + 5 % CO₂ pendant cette période et suivie par une réoxygénation de 30 mn.
Les composés à tester sont mis en incubation 15 mn avant et pendant la durée de l'hypoxie.
Les contractions isovolumétriques sont enregistrées par l'intermédiaire d'un ballonet en polyéthylène relié à un capteur de pression (P23-GOULD) introduit dans le ventricule gauche et gonflé de manière à obtenir une pression diastolique comprise entre 5 et 10 mm de mercure.

### Résultats

| **Effet protecteur des produits des exemples 2 et 3 sur un coeur isolé de rat soumis à une hypoxie-réoxygénation** | | | |
|---|---|---|---|
| | **SOLVANT H**_{**2**}**O (n=7)** | **Exemple 2 8.10**^{**-7**} **M (n=4)** | **Exemple 3 8.10**^{**-7**} **M (n=5)** |
| % PVG à 30 min DE REOXYGENATION | 45,2 ± 10 | 95,0 ± 1,9 | 85,4 ± 5,8 |

| AMPLITUDE CONTRACTURE (mmHg) | | | |
|---|---|---|---|
| à 10 min d'hypoxie | 21,1 ± 3,2 | 12,5 ± 2,2 | 20,8 ± 4,6 |
| à 30 min d'hypoxie | 35,1 ± 6,6 | 12,5 ± 3,0 | 26,0 ± 5,1 |
| à 60 min d'hypoxie | 31,4 ± 6,8 | 11,5 ± 2,6 | 25,6 ± 2,9 |
| à 15 min de réoxygénation | 20,9 ± 5,9 | 5,5 ± 4,3 | 9,6 ± 2,4 |
| à 30 min de réoxygénation | 12,3 ± 4,3 | 0,5 ± 0,5 | 1,6 ± 1,0 |

Ces résultats montrent que les composés de la présente invention limitent également le développement de la contracture au cours de l'hypoxie et permettent une meilleure récupération fonctionnelle au cours de la réoxygénation.

### CONCLUSION

On montre donc par ces études que les composés de la présente invention protègent efficacement le myocarde vis à vis d'une surcharge en calcium intra-cellulaire (intoxication à la ouabaïne) ou vis à vis d'une hypoxie-réoxygénation.

## Revendications (Revendications pour l'(les) Etat(s) contractant(s) suivant(s): AT, BE, CH, DE, DK, FR, GB, IT, LI, LU, NL, PT, SE)

1. Les composés de formule générale (I) : 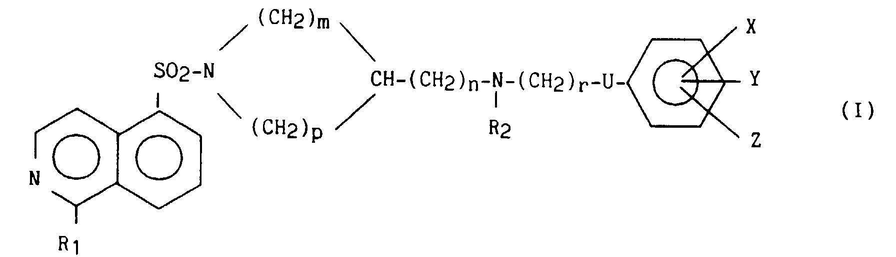 dans laquelle :
n est 0 ou un nombre entier de 1 à 3,
m et p sont des nombres entiers de 1 à 4 étant entendu que la somme m+p est 2, 3, 4 ou 5,
r est un nombre entier de 1 à 6,
R₁ représente un atome d'hydrogène, un groupement hydroxy ou un atome de chlore,
R₂ représente :
- un atome d'hydrogène,
- un groupement formyle,
- un groupement -A, -CO-A, -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué, avec q signifiant 0 ou un nombre entier de 1 à 4,
- un groupement -CO-phényl ou un groupement -CO-phényl substitué,
- un groupement -CO-O-phényl ou un groupement -CO-O-phényl substitué,
- un groupement -CO-NR₄R₅
où R₄, R₅ : - identiques ou différents, représentent un atome d'hydrogène ou un groupement choisi parmi alkyle inférieur, alkényl inférieur, alkynyl inférieur, phényle, phénylalkyle inférieur,
- soit forment avec l'atome d'azote qui les porte un cycle saturé de 4 à 7 chaînons,
X, Y, Z identiques ou différents, représentent chacun indépendamment l'un de l'autre un atome d'hydrogène, un atome d'halogène, ou un groupement choisi parmi alkyle inférieur, alcoxy inférieur, nitro, amino, cyano, acétamido, carboxamido, ou X et Y, ou X et Z forment ensemble, avec les 2 atomes de carbone du noyau phényle qui les portent, un cycle furanique, dihydrofuranique, ou benzénique,
U représente un atome d'oxygène, un atome de soufre ou un groupement choisi parmi :
carbonyl, sulfinyl, sulfonyl, -NH-CO-, -CO-NH-, -O-(CH₂)_{r′}-O- avec r′ signifiant un nombre entier égal à 2 ou 3, -(CH₂)_{r˝}-O-(CH₂)_{r‴}- avec r˝ et r‴ signifiant des nombres entiers égaux à 1 ou 2, et 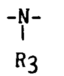
où
R₃ représente :
- un atome d'hydrogène,
- un groupement formyle,
- un groupement -A, -CO-A, ou -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué avec q signifiant 0 ou un nombre entrer de 1 à 4,
- un groupement -CO-phényl ou un groupement -CO-phényl substitué,
- un groupement -CO-O-phényl ou un groupement -CO-O-phényl substitué,
- un groupement -CO-NR₄R₅ avec R₄ et R₅ tels que définis précédemment,
étant entendu que le terme "substitué" affectant les groupements -(CH₂)_{q}-phényl, -CO-phényl, ou -CO-O-phényl, signifie que ces groupements peuvent être substitués par un ou plusieurs radicaux choisis parmi : alkyle inférieur, alcoxy inférieur, hydroxy, atome d'halogène, et trifluorométhyle,
- que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkynyle inférieur" désignent des groupements insaturés linéaires ou ramifiés contenant de 2 à 6 atomes de carbone, et
- que le terme "cycloalkyl" désigne un cycle carboné saturé contenant de 3 à 8 chaînons,
leurs éventuels isomères optiques ainsi que, le cas échéant, leurs sels d'addition à un acide pharmaceutiquement acceptable.

2. Les composés de formule (I) selon la revendication 1 dans lesquels m et p représentent chacun 2, ainsi que leurs sels d'addition avec un acide pharmaceutiquement acceptable.

3. Les composés de formule (I) selon la revendication 1 dans lesquels U représente un atome d'oxygène ou un groupement carbonyl,
leurs éventuels isomères optiques, ainsi que leurs sels d'addition à un acide pharmaceutiquement acceptable.

4. Le composé selon la revendication 1 qui est la 1-[(isoquinoléin-5 yl)sulfonyl] 4-{N(méthyl) N-[4-(p-fluorophénoxy)butyl]amino}pipéridine,
ainsi que ses sels d'addition à un acide pharmaceutiquement acceptable.

5. Le composé selon la revendication 1 qui est la 1-[(isoquinoléin-5 yl)sulfonyl] 4-{N-(méthyl) N-[4-(oxo) 4-(p-fluorophényl)butyl]amino} pipéridine,
ainsi que ses sels d'addition à un acide pharmaceutiquement acceptable.

6. Le composé selon la revendication 1 qui est la 1-[(isoquinoléin-5 yl)sulfonyl] 4-{{N-(méthyl) N-[3-(p-fluorophénoxy)propyl]amino}méthyl} pipéridine,
ainsi que ses sels d'addition à un acide pharmaceutiquement acceptable.

7. Le composé selon la revendication 1 qui est la 1-[(isoquinoléin-5 yl)sulfonyl] 4-{N-(méthyl) N-[5-(p-fluorophénoxy)pentyl]amino} pipéridine,
ainsi que ses sels d'addition avec un acide pharmaceutiquement acceptable.

8. Le composé de la revendication 1 qui est la 1-[(isoquinoléin-5 yl)sulfonyl] 4-{N-méthyl N-[4-(p-fluorophényl) 4-oxo butyl]amino méthyl}pipéridine ainsi que ses sels d'addition avec un acide pharmaceutiquement acceptable.

9. Le procédé de préparation des composés de formule générale (I) selon la revendication 1, caractérisé en ce que :
- on fait réagir un composé de formule (II) : 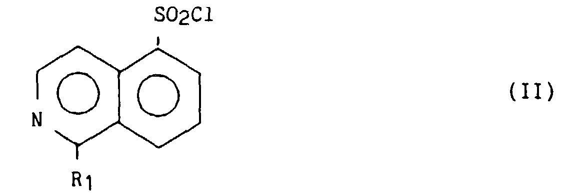 dans laquelle R₁ a la même signification que dans la formule générale (I),
- soit avec un composé de formule (III) : 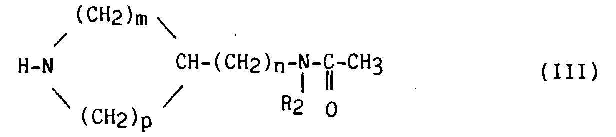 dans laquelle R₂, n, m et p ont la même signification que dans la formule générale (I),
pour obtenir, après hydrolyse acide, un composé de formule (IV) : 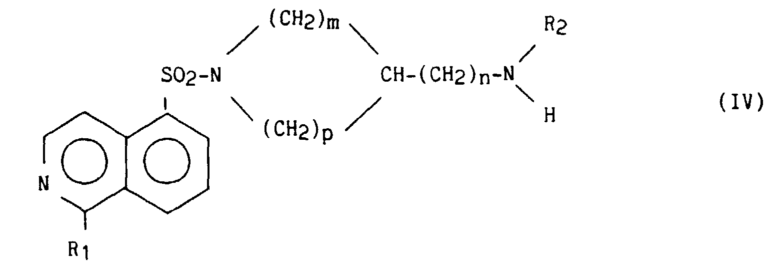 dans laquelle R₁, R₂, n, m et p sont tels que définis précédemment, que l'on fait réagir avec un composé de formule (V) : 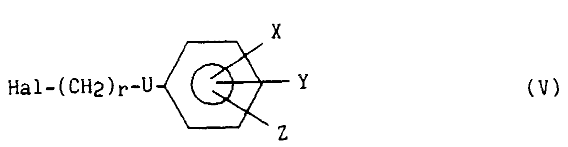 dans laquelle X, Y, Z, U et r ont la même signification que dans la formule générale (I) et Hal représente un atome d'halogène, pour obtenir un composé de formule (I) : 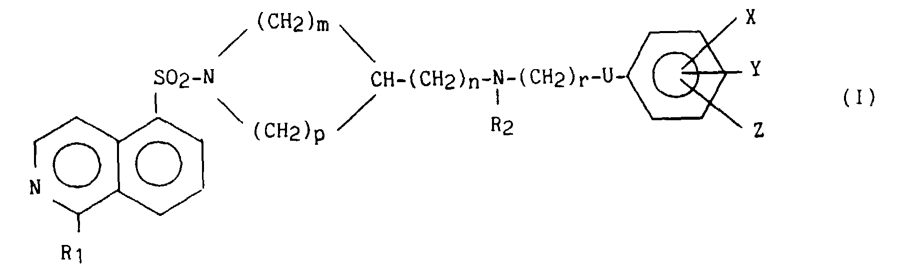 dans laquelle R₁, R₂, U, X, Y, Z, n, m, p et r sont tels que définis dans la formule générale (I),
- soit avec un composé de formule (VI) : 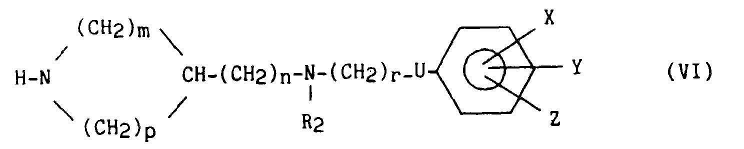 dans laquelle R₂, U, X, Y, Z, n, m, p et r sont tels que définis précédemment, pour obtenir un composé de formule (I), dont on sépare, le cas échéant, les isomères, et que l'on salifie, si on le désire, avec un acide pharmaceutiquement acceptable,
composé de formule (I) que l'on purifie, si on le désire, par une technique de cristallisation et/ou de chromatographie.

10. Composition pharmaceutique contenant comme principe actif au moins un composé selon les revendications 1 à 8 en combinaison avec un ou plusieurs excipients ou véhicules inertes, non toxiques, et pharmaceutiquement acceptables.

11. Composition pharmaceutique selon la revendication 10, utilisable dans le traitement et la prévention des affections dues ou reliées à des phénomènes de souffrance tissulaire et notamment dans le traitement et la prévention de l'ischémie myocardique et périphérique, des maladies vasculaires, de l'hypertension artérielle, de l'insuffisance cardiaque, des troubles du rythme cardiaque, des troubles liés au vieillissement artériel et à l'athérosclérose, des pathologies métaboliques constituant un facteur de risque cardiovasculaire, des thromboses, des affections relatives aux muscles lisses bronchiques, digestifs, urinaires, et utérins, des situations de souffrance tissulaire liées au vieillissement, à l'ischémie, à l'inflammation ou à une prolifération cellulaire, et également utilisable pour la prévention des resténoses ou thromboses vasculaires après pontage, dilatation vasculaire notamment coronaire, ou d'autres formes de reperméabilisation vasculaire.

## Revendications (Revendications pour l'(les) Etat(s) contractant(s) suivant(s): ES, GR)

1. Le procédé de préparation des composés de formule générale (I) : 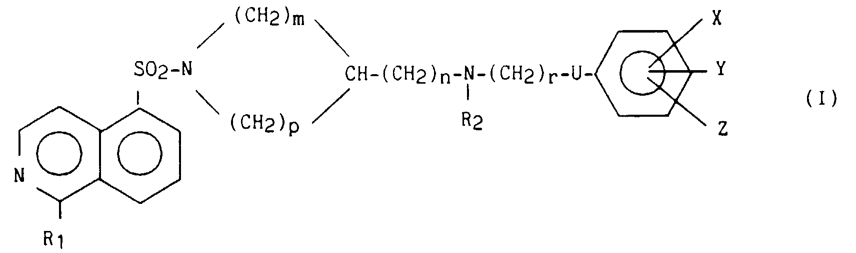 dans laquelle :
n est 0 ou un nombre entier de 1 à 3,
m et p sont des nombres entiers de 1 à 4 étant entendu que la somme m+p est 2, 3, 4 ou 5,
r est un nombre entier de 1 à 6,
R₁ représente un atome d'hydrogène, un groupement hydroxy ou un atome de chlore,
R₂ représente :
- un atome d'hydrogène,
- un groupement formyle,
- un groupement -A, -CO-A, -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué, avec q signifiant 0 ou un nombre entier de 1 à 4,
- un groupement -CO-phényl ou un groupement -CO-phényl substitué,
- un groupement -CO-O-phényl ou un groupement -CO-O-phényl substitué,
- un groupement -CO-NR₄R₅
où R₄, R₅ : - identiques ou différents, représentent un atome d'hydrogène ou un groupement choisi parmi alkyle inférieur, alkényl inférieur, alkynyl inférieur, phényle, phénylalkyle inférieur,
- soit forment avec l'atome d'azote qui les porte un cycle saturé de 4 à 7 chaînons,
X, Y, Z identiques ou différents, représentent chacun indépendamment l'un de l'autre un atome d'hydrogène, un atome d'halogène, ou un groupement choisi parmi alkyle inférieur, alcoxy inférieur, nitro, amino, cyano, acétamido, carboxamido, ou X et Y, ou X et Z forment ensemble, avec les 2 atomes de carbone du noyau phényle qui les portent, un cycle furanique, dihydrofuranique, ou benzénique,
U représente un atome d'oxygène, un atome de soufre ou un groupement choisi parmi :
carbonyl, sulfinyl, sulfonyl, -NH-CO-, -CO-NH-, -O-(CH₂)_{r'}-O- avec r' signifiant un nombre entier égal à 2 ou 3, -(CH₂)_{r"}-O-(CH₂)_{r"'}- avec r" et r"' signifiant des nombres entiers égaux à 1 ou 2, et 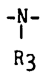
où
R₃ représente :
- un atome d'hydrogène,
- un groupement formyle,
- un groupement -A, -CO-A, ou -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué avec q signifiant O ou un nombre entier de 1 à 4,
- un groupement -CO-phényl ou un groupement -CO-phényl substitué,
- un groupement -CO-O-phényl ou un groupement -CO-O-phényl substitué,
- un groupement -CO-NR₄R₅ avec R₄ et R₅ tels que définis précédemment,
[étant entendu que le terme "substitué" affectant les groupements -(CH₂)_{q}-phényl, -CO-phényl, ou -CO-O-phényl, signifie que ces groupements peuvent être substitués par un ou plusieurs radicaux choisis parmi : alkyle inférieur, alcoxy inférieur, hydroxy, atome d'halogène, et trifluorométhyle,
- que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkynyle inférieur" désignent des groupements insaturés linéaires ou ramifiés contenant de 2 à 6 atomes de carbone, et
- que le terme "cycloalkyl" désigne un cycle carboné saturé contenant de 3 à 8 chaînons, ]
de leurs éventuels isomères optiques ainsi que, le cas échéant, de leurs sels d'addition à un acide pharmaceutiquement acceptable,
caractérisé en ce que :
- on fait réagir un composé de formule (II) : 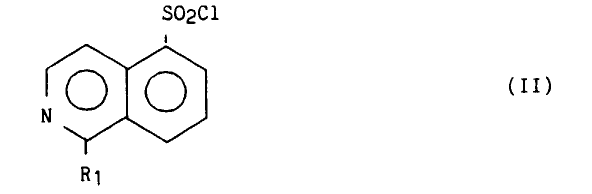 dans laquelle R₁ a la même signification que dans la formule générale (I),
a) soit avec un composé de formule (III) : 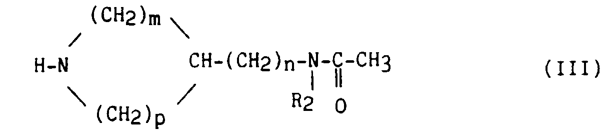 dans laquelle R₂, n, m et p ont la même signification que dans la formule générale (I),
pour obtenir, après hydrolyse acide, un composé de formule (IV) : 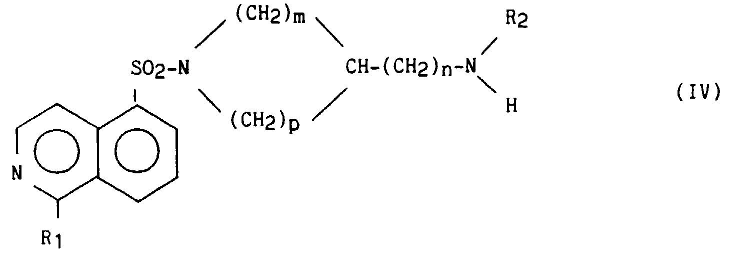 dans laquelle R₁, R₂, n, m et p sont tels que définis précédemment, que l'on fait réagir avec un composé de formule (V) : 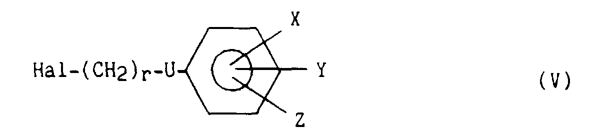 dans laquelle X, Y, Z, U et r ont la même signification que dans la formule générale (I) et Hal représente un atome d'halogène ;
b) soit avec un composé de formule (VI) : 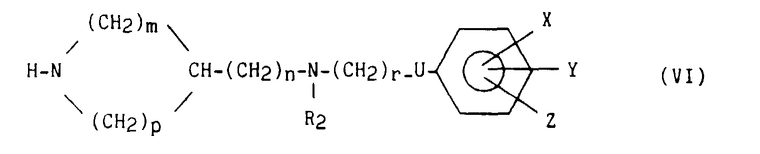 dans laquelle R₂, U, X, Y, Z, n, m, p et r sont tels que définis précédemment,
- et le cas échéant, on sépare les isomères du composé I obtenu et/ou, si on le désire, on traite par un acide pharmaceutiquement acceptable pour donner les sels d'addition acides correspondants.

## Patentansprüche (Patentansprüche für folgende(n) Vertragsstaat(en): AT, BE, CH, DE, DK, FR, GB, IT, LI, LU, NL, PT, SE)

1. Verbindungen der allgemeinen Formel (I): 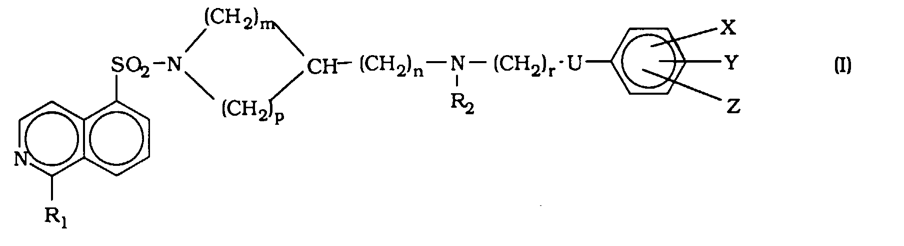 in der:
n 0 oder eine ganze Zahl mit einem Wert von 1 bis 3,
m und p ganze Zahlen mit Werten von 1 bis 4, mit der Maßgabe, daß die Summe von m+p 2, 3, 4 oder 5 beträgt,
r eine ganze Zahl mit einem Wert von 1 bis 6,
R₁ ein Wasserstoffatom, eine Hydroxylgruppe oder ein Chloratom, R₂:
- ein Wasserstoffatom,
- eine Formylgruppe,
- eine Gruppe -A, -CO-A, -CO-O-A, worin A eine Niedrigalkylgruppe, Niedrigalkenylgruppe, Niedrigalkinylgruppe, Cycloalkylgruppe oder Niedrigcycloalkylalkylgruppe darstellt,
- eine -(CH₂)_{q}-Phenylgruppe oder eine substituierte -(CH₂)_{q}-Phenylgruppe, worin q O oder eine ganze Zahl mit einem Wert von 1 bis 4 darstellt,
- eine -CO-Phenylgruppe oder eine substituierte -CO-Phenylgruppe,
- eine -CO-O-Phenylgruppe oder eine substituierte -CO-O-Phenylgruppe,
- eine Gruppe -CO-NR₄R₅
in der R₄, R₅, - die gleichartig oder verschieden sein können, ein Wasserstoffatom oder eine Gruppe ausgewählt aus Niedrigalkylgruppen, Niedrigalkenylgruppen, Niedrigalkinylgruppen, Phenylgruppen und Niedrigphenylalkylgruppen darstellen,
- oder zusammen mit dem sie tragenden Stickstoffatom einen gesättigten Ring mit 4 bis 7 Kettengliedern bilden,
X, Y, Z, die gleichartig oder verschieden sein können, jeweils unabhängig voneinander ein Wasserstoffatom, ein Halogenatom oder eine Gruppe ausgewählt aus Niedrigalkylgruppen, Niedrigalkoxygruppen, Nitrogruppen, Aminogruppen, Cyanogruppen, Acetamidogruppen oder Carboxamidogruppen oder X und Y oder X und Z gemeinsam mit den beiden Kohlenstoffatomen des sie tragenden Phenylkerns einen Furan-, Dihydrofuran- oder Benzolring und
U ein Sauerstoffatom, ein Schwefelatom oder eine Gruppe ausgewählt aus: Carbonyl, Sulfinyl, Sulfonyl, -NH-CO-. -CO-NH-, -O-(CH₂)_{r'}-O-, worin r' eine ganze Zahl mit einem Wert von 2 oder 3 darstellt, -(CH₂)_{r"}-O-(CH₂)r_{"'}-, worin r" und r"' ganze Zahlen mit Wert von 1 oder 2 darstellen, und 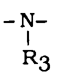
in der R₃:
- ein Wasserstoffatom,
- eine Formylgruppe,
- eine Gruppe -A, -CO-A oder -CO-O-A, worin A eine Niedrigalkylgruppe, Niedrigalkenylgruppe, Niedrigalkinylgruppe, Cycloalkylgruppe oder Niedrigcycloalkylalkylgruppe darstellt,
- eine -(CH₂)_{q}-Phenylgruppe oder eine substituierte -(CH₂)_{q}-Phenylgruppe, worin q 0 oder eine ganze Zahl mit einem Wert von 1 bis 4 darstellt,
- eine -CO-Phenylgruppe oder eine substituierte -CO-Phenylgruppe,
- eine -CO-O-Phenylgruppe oder eine substituierte -CO-O-Phenylgruppe oder
- eine -CO-NR₄R₅-Gruppe, in der R₄ und R₅ die oben angegebenen Bedeutungen besitzen, darstellt,
bedeuten,
wobei der Begriff"substituiert" bezüglich der -(CH₂)_{q}-Phenyl-, -CO-Phenyl- oder -CO-O-Phenylgruppen bedeutet, daß diese Gruppen durch einen oder mehrere Reste ausgewählt aus Niedrigalkylgruppen, Niedrigalkoxygruppen, Hydroxylgruppen, Halogenatomen und Trifluormethylgruppen substituiert sein können,
- die Begriffe "Niedrigalkyl" und "Niedrigalkoxy" bedeuten, daß die gesättigten geradkettigen oder verzweigten Kohlenstoffgruppen 1 bis 6 Kohlenstoffatome enthalten,
- die Begriffe "Niedrigalkenyl" und "Niedrigalkinyl" bedeuten, daß die ungesättigten geradkettigen oder verzweigten Gruppen 2 bis 6 Kohlenstoffatome enthalten, und
- der Begriff "Cycloalkyl" für einen gesättigten Kohlenstoffring mit 3 bis 8 Kohlenstoffatomen steht,
deren eventuelle optische Isomere sowie gegebenenfalls deren Additionssalze mit einer pharmazeutisch annehmbaren Säure.

2. Verbindungen der Formel (I) nach Anspruch 1, worin m und p jeweils 2 bedeuten, sowie deren Additionssalze mit einer pharmazeutisch annehmbaren Säure.

3. Verbindungen der Formel (I) nach Anspruch 1, worin U ein Sauerstoffatom oder eine Carbonylgruppe bedeutet, deren mögliche optische Isomeren sowie deren Additionssalze mit einer pharmazeutisch annehmbaren Säure.

4. Verbindung nach Anspruch 1, nämlich 1-[(Isochinolin-5-yl)-sulfonyl]-4-(N-(methyl)-N-[4-(p-fluorphenoxy]-butyl]-amino)-piperidin, sowie dessen Additionssalze mit einer pharmazeutisch annehmbaren Säure.

5. Verbindung nach Anspruch 1, nämlich 1-[(Isochinolin-5-yl)-sulfonyl]-4-(N-(methyl)-N-[4-(oxo)-4-(p-fluorphenyl)-butyl]-amino)-piperidin, und dessen Additionssalze mit einer pharmazeutisch annehmbaren Säure.

6. Verbindung nach Anspruch 1, nämlich 1-[(Isochinolin-5-yl)-sulfonyl]-4-{{N-(methyl)-N-[3-(p-fluorphenoxy]-propyl]-amino}-methyl}-piperidin, sowie dessen Additionssalze mit einer pharmazeutisch annehmbaren Säure.

7. Verbindung nach Anspruch 1, nämlich 1-[(Isochinolin-5-yl)-sulfonyl]-4-{N-(methyl)-N-[5-(p-fluorphenoxy)-pentyl]-amino}-piperidin, sowie dessen Additionssalze mit einer pharmazeutisch annehmbaren Säure.

8. Verbindung nach Anspruch 1, nämlich 1-[(Isochinolin-5-yl)-sulfonyl]-4-(N-methyl-N-[4-[p-fluorphenyl)-4-oxo-butyl]-aminomethyl)-piperidin, sowie dessen Additionssalze mit einer pharmazeutisch annehmbaren Säure.

9. Verfahren zur Herstellung der Verbindungen der allgemeinen Formel (I) nach Anspruch 1, **dadurch gekennzeichnet**, daß man:
- eine Verbindung der Formel (II): 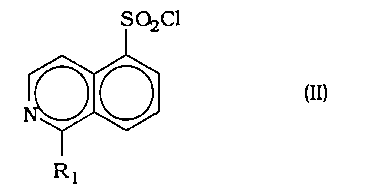 in der R₁ die bezüglich der allgemeinen Formel (I) angegebenen Bedeutungen besitzt,
- entweder mit einer Verbindung der Formel (III): 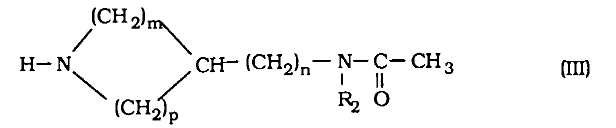 in der R₂, n, m und p die bezüglich der allgemeinen Formel (I) angegebenen Bedeutungen besitzen, umsetzt, so daß man nach der sauren Hydrolyse eine Verbindung der Formel (IV) erhält: 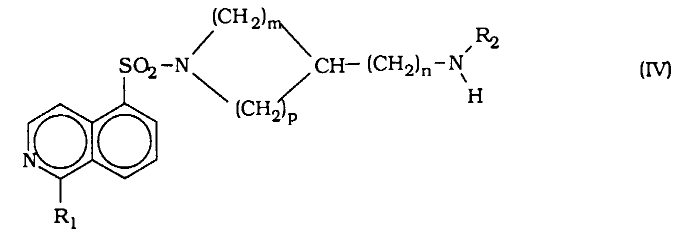 in der R₁, R₂, n, m und p die oben angegebenen Bedeutungen besitzen, welche man mit einer Verbindung der Formel (V) umsetzt: 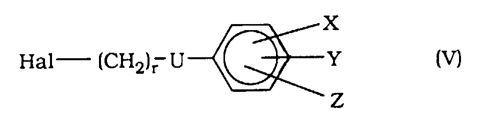 in der X, Y, Z, U und r die bezüglich der allgemeinen Formel (I) angegebenen Bedeutungen besitzen und Hal ein Halogenatom darstellt, zur Bildung einer Verbindung der Formel (I): 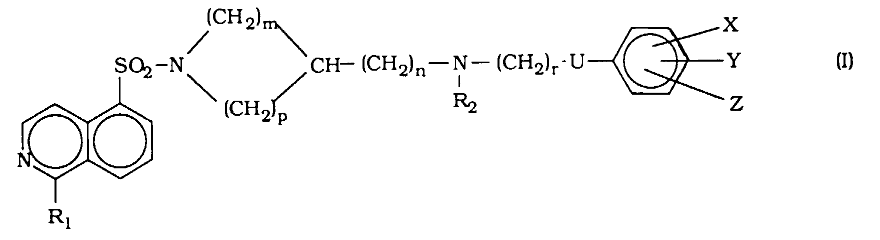 in der R₁, R₂, U, X, Y, Z, n, m, p und r die bezüglich der allgemeinen Formel (I) angegebenen Bedeutungen besitzen,
- oder mit einer Verbindung der Formel (VI): 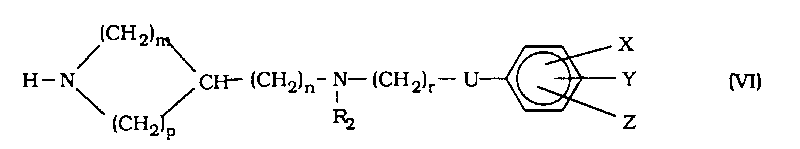 in der R₂, U, X, Y, Z, n, m, p und r die oben angegebenen Bedeutungen besitzen, umsetzt zur Bildung einer Verbindung der Formel (I), die man gegebenenfalls in ihre Isomeren auftrennt und gewünschtenfalls mit einer pharmazeutisch annehmbaren Säure in ein Salz überführt,
welche Verbindung der Formel (I) man gewünschtenfalls mit Hilfe einer Kristallisations- und/oder Chromatographie-Methode reinigt.

10. Pharmazeutische Zubereitung enthaltend als Wirkstoff mindestens eine Verbindung nach den Ansprüchen 1 bis 8 in Kombination mit einem oder mehreren interten, nichttoxischen und pharmazeutisch annehmbaren Trägermaterialien oder Bindemitteln.

11. Pharmazeutische Zubereitung nach Anspruch 10 zur Verwendung bei der Behandlung und der Vorbeugung von Erkrankungen, die eine Folge von oder verknüpft sind mit Phänomenen von Gewebeerkrankungen und insbesondere bei der Behandlung und der Vorbeugung von Myokard- und peripherer Ischämie, von Gefäßerkrankungen, der arteriellen Hypertension, von Herzinsuffizienzen, von Störungen des Herzrhythmus, von mit dem Altern von Arterien und der Atherosklerose verknüpften Störungen, von Stoffwechselerkrankungen, die einen kardiovaskulären Risikofaktor darstellen, von Thrombosen, von Erkrankungen betreffend die glatten Muskeln der Bronchien, des Verdauungstrakts, des Harntrakts und des Uterus, von Situationen von Gewebeerkrankungen, die mit dem Altern, der Ischämie, der Entzündung oder einer Zellwucherung verknüpft sind und die auch zur Vorbeugung von Gefäßrestenosen oder -thrombosen nach Bypass-Operationen, der Gefäßerweiterung, insbesondere von Koronargefäßen, oder von anderen Formen der Durchgängigmachung von Gefäßen verwendbar sind.

## Patentansprüche (Patentansprüche für folgende(n) Vertragsstaat(en): ES, GR)

1. Verfahren zur Herstellung der Verbindungen der allgemeinen Formel (I): 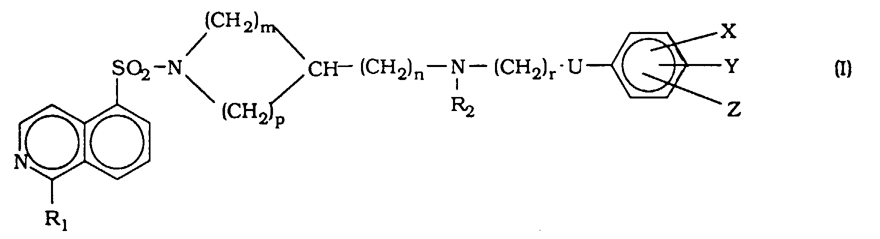 in der:
n 0 oder eine ganze Zahl mit einem Wert von 1 bis 3,
m und p ganze Zahlen mit Werten von 1 bis 4, mit der Maßgabe, daß die Summe von m+p 2, 3, 4 oder 5 beträgt,
r eine ganze Zahl mit einem Wert von 1 bis 6,
R₁ ein Wasserstoffatom, eine Hydroxylgruppe oder ein Chloratom, R₂:
- ein Wasserstoffatom,
- eine Formylgruppe,
- eine Gruppe -A, -CO-A, -CO-O-A, worin A für eine Niedrigalkylgruppe, Niedrigalkenylgruppe, Niedrigalkinylgruppe, Cycloalkylgruppe oder Niedrigcycloalkylalkylgruppe darstellt,
- eine -(CH₂)_{q}-Phenylgruppe oder eine substituierte -(CH₂)_{q}-Phenylgruppe, worin q 0 oder eine ganze Zahl mit einem Wert von 1 bis 4 darstellt,
- eine -CO-Phenylgruppe oder eine substituierte -CO-Phenylgruppe,
- eine -CO-O-Phenylgruppe oder eine substituierte -CO-O-Phenylgruppe,
- eine Gruppe -CO-NR₄R₅
in der R₄, R₅, - die gleichartig oder verschieden sein können, ein Wasserstoffatom oder eine Gruppe ausgewählt aus Niedrigalkylgruppen, Niedrigalkenylgruppen, Niedrigalkinylgruppen, Phenylgruppen und Niedrigphenylalkylgruppen darstellen,
- oder zusammen mit dem sie tragenden Stickstoffatom einen gesättigten Ring mit 4 bis 7 Kettengliedern bilden,
X, Y, Z, die gleichartig oder verschieden sein können, jeweils unabhängig voneinander ein Wasserstoffatom, ein Halogenatom oder eine Gruppe ausgewählt aus Niedrigalkylgruppen, Niedrigalkoxygruppen, Nitrogruppen, Aminogruppen, Cyanogruppen, Acetamidogruppen oder Carboxamidogruppen oder X und Y oder X und Z gemeinsam mit den beiden Kohlenstoffatomen des sie tragenden Phenylkerns einen Furan-, Dihydrofuran- oder Benzolring und
U ein Sauerstoffatom, ein Schwefelatom oder eine Gruppe ausgewählt aus: Carbonyl, Sulfinyl, Sulfonyl, -NH-CO-, -CO-NH-, -O-(CH₂)_{r'}-O-, worin r' eine ganze Zahl mit einem Wert von 2 oder 3 darstellt, -(CH₂)_{r"}-O-(CH₂)r_{"'}-, worin r" und r"' ganze Zahlen mit Wert von 1 oder 2 darstellen, und 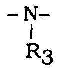
in der R₃:
- ein Wasserstoffatom,
- eine Formylgruppe,
- eine Gruppe -A, -CO-A oder -CO-O-A, worin A eine Niedrigalkylgruppe, Niedrigalkenylgruppe, Niedrigalkinylgruppe, Cycloalkylgruppe oder Niedrigcycloalkylalkylgruppe darstellt,
- eine -(CH₂)_{q}-Phenylgruppe oder eine substituierte -(CH₂)_{q}-Phenylgruppe, worin q 0 oder eine ganze Zahl mit einem Wert von 1 bis 4 darstellt,
- eine -CO-Phenylgruppe oder eine substituierte -CO-Phenylgruppe,
- eine -CO-O-Phenylgruppe oder eine substituierte -CO-O-Phenylgruppe oder
- eine -CO-NR₄R₅-Gruppe, in der R₄ und R₅ die oben angegebenen Bedeutungen besitzen, darstellt,
bedeuten,
[wobei der Begriff "substituiert" bezüglich der -(CH₂)_{q}-Phenyl-, -CO-Phenyl- oder -CO-O-Phenylgruppen bedeutet, daß diese Gruppen durch einen oder mehrere Reste ausgewählt aus Niedrigalkylgruppen, Niedrigalkoxygruppen, Hydroxylgruppen, Halogenatomen und Trifluormethylgruppen substituiert sein können,
- die Begriffe "Niedrigalkyl" und "Niedrigalkoxy" bedeuten, daß die gesättigten geradkettigen oder verzweigten Kohlenstoffgruppen 1 bis 6 Kohlenstoffatome enthalten,
- die Begriffe "Niedrigalkenyl" und "Niedrigalkinyl" bedeuten, daß die ungesättigten geradkettigen oder verzweigten Gruppen 2 bis 6 Kohlenstoffatome enthalten, und
- der Begriff "Cycloalkyl" für einen gesättigten Kohlenstoffring mit 3 bis 8 Kohlenstoffatomen steht,]
und von deren eventuellen optischen Isomeren sowie gegebenenfalls von deren Additionssalzen mit einer pharmazeutisch annehmbaren Säure, **dadurch gekennzeichnet**, daß man:
- eine Verbindung der Formel (II): 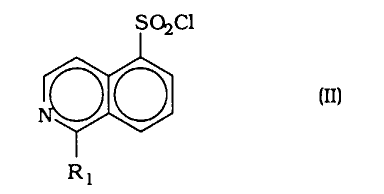 in der R₁ die bezüglich der allgemeinen Formel (I) angegebenen Bedeutungen besitzt,
a) entweder mit einer Verbindung der Formel (III): 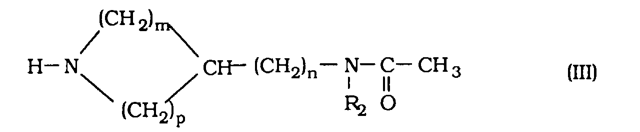 in der R₂, n, m und p die bezüglich der allgemeinen Formel (I) angegebenen Bedeutungen besitzen, umsetzt, so daß man nach der sauren Hydrolyse eine Verbindung der Formel (IV) erhält: 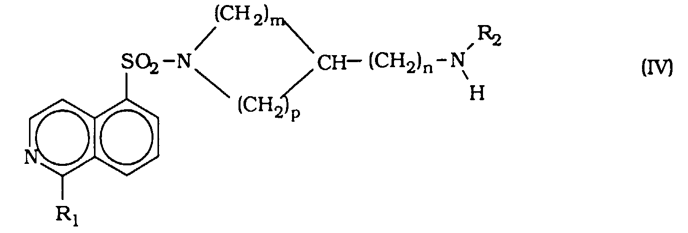 in der R₁, R₂, n, m und p die oben angegebenen Bedeutungen besitzen, welche man mit einer Verbindung der Formel (V) umsetzt: 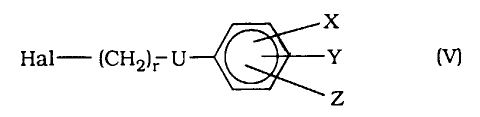 in der X, Y, Z, U und r die bezüglich der allgemeinen Formel (I) angegebenen Bedeutungen besitzen und Hal ein Halogenatom darstellt,
b) oder mit einer Verbindung der Formel (VI): 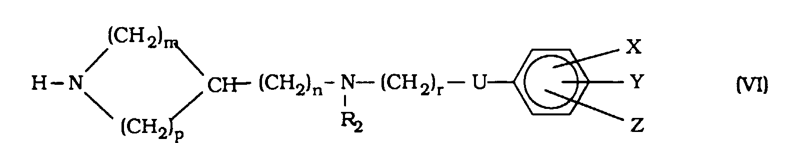 in der R₂, U, X, Y, Z, n, m, p und r die oben angegebenen Bedeutungen besitzen, umsetzt
- und gegebenenfalls die Isomeren der erhaltenen Verbindung I trennt und/ oder gewünschtenfalls mit einer pharmazeutisch annehmbaren Säure behandelt zur Bildung der entsprechenden Säureadditionssalze.

## Claims (Claims for the following Contracting State(s): AT, BE, CH, DE, DK, FR, GB, IT, LI, LU, NL, PT, SE)

1. Compounds of the general formula (I): 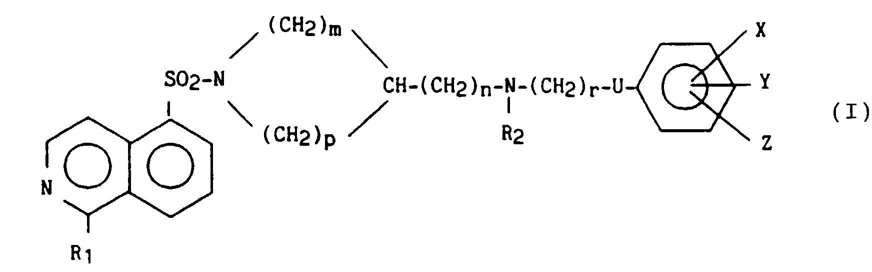 in which:
n is 0 or an integer from 1 to 3,
m and p are integers from 1 to 4 with the proviso that the sum of m+p is 2, 3, 4 or 5,
r is an integer from 1 to 6,
R₁ represents a hydrogen atom, a hydroxy group or a chlorine atom,
R₂ represents:
- a hydrogen atom,
- a formyl group,
- a group -A, -CO-A or -CO-O-A, wherein A represents a lower alkyl, lower alkenyl, lower alkynyl, cycloalkyl or cycloalkyl-lower alkyl group,
- a -(CH₂)_{q}-phenyl group or a substituted -(CH₂)_{q}-phenyl group, wherein q represents 0 or an integer from 1 to 4,
- a -CO-phenyl group or a substituted -CO-phenyl group,
- a -CO-O-phenyl group or a substituted -CO-O-phenyl group, or
- a group -CO-NR₄R₅, wherein R₄ and R₅
- which may be identical or different, each represent a hydrogen atom or a group selected from lower alkyl, lower alkenyl, lower alkynyl, phenyl and phenyl-lower alkyl, or
- together with the nitrogen atom carrying them, form a saturated ring having from 4 to 7 ring members,
X, Y and Z, which may be identical or different, each independently of the others represents a hydrogen atom, a halogen atom, or a group selected from lower alkyl, lower alkoxy, nitro, amino, cyano, acetamido and carboxamido, or X and Y, or X and Z, together form, with the two carbon atoms of the phenyl nucleus carrying them, a furan, dihydrofuran or benzene ring,
U represents an oxygen atom, a sulphur atom or a group selected from: carbonyl, sulphinyl, sulphonyl, -NH-CO-, -CO-NH-, -O-(CH₂)_{r'}-o- wherein r' represents an integer 2 or 3, -(CH₂)_{r"}-O-(CH₂)_{r"'}- wherein r" and r'" each represent integers 1 or 2, and 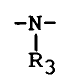 wherein R₃ represents:
- a hydrogen atom,
- a formyl group,
- a group -A, -CO-A or -CO-O-A, wherein A represents a lower alkyl, lower alkenyl, lower alkynyl, cycloalkyl or cycloalkyl-lower alkyl group,
- a -(CH₂)_{q}-phenyl group or a substituted -(CH₂)_{q}-phenyl group, wherein q represents 0 or an integer from 1 to 4,
- a -CO-phenyl group or a substituted -CO-phenyl group,
- a -CO-O-phenyl group or a substituted -CO-O-phenyl group, or
- a group -CO-NR₄R₅, wherein R₄ and R₅ are as defined above,
wherein the term "substituted" relating to the groups -(CH₂)_{q}-phenyl, -CO-phenyl and -CO-O-phenyl means that those groups may be substituted by one or more radicals selected from: lower alkyl, lower alkoxy, hydroxy, halogen and trifluoromethyl,
- the terms "lower alkyl" and "lower alkoxy" indicate straight-chained or branched saturated carbon-containing groups containing from 1 to 6 carbon atoms,
- the terms "lower alkenyl" and "lower alkynyl" designate straight-chained or branched unsaturated groups containing from 2 to 6 carbon atoms, and
- the term "cycloalkyl" designates a saturated carbon-containing ring containing from 3 to 8 ring members,
the possible optical isomers thereof and, where applicable, the addition salts thereof with a pharmaceutically acceptable acid.

2. Compounds of formula (I) according to claim 1 in which m and p each represent 2, as well as the addition salts thereof with a pharmaceutically acceptable acid.

3. Compounds of formula (I) according to claim 1 in which U represents an oxygen atom or a carbonyl group, the possible optical isomers thereof, as well as the addition salts thereof with a pharmaceutically acceptable acid.

4. Compound according to claim 1 which is 1-(isoquinolin-5-ylsulphonyl)-4-{N-methyl-N-[4-(p-fluorophenoxy)butyl]amino}piperidine, as well as the addition salts thereof with a pharmaceutically acceptable acid.

5. Compound according to claim 1 which is 1-(isoquinolin-5-ylsulphonyl)-4-(N-methyl-N-[4-oxo-4-(p-fluorophenyl)butyl]amino)piperidine, as well as the addition salts thereof with a pharmaceutically acceptable acid.

6. Compound according to claim 1 which is 1-(isoquinolin-5-ylsulphonyl)-4-{{N-methyl-N-[3-(p-fluorophenoxy)propyl]amino}methyl}piperidine, as well as the addition salts thereof with a pharmaceutically acceptable acid.

7. Compound according to claim 1 which is 1-(isoquinolin-5-ylsulphonyl)-4-{N-methyl-N-[5-(p-fluorophenoxy)pentyl]amino}piperidine, as well as the addition salts thereof with a pharmaceutically acceptable acid.

8. Compound according to claim 1 which is 1-(isoquinolin-5-ylsulphonyl)-4-{N-methyl-N-[4-(p-fluorophenyl)-4-oxobutyl]aminomethyl}piperidine, as well as the addition salts thereof with a pharmaceutically acceptable acid.

9. Process for the preparation of the compounds of the general formula (I) according to claim 1, characterised in that:
- a compound of formula (II): 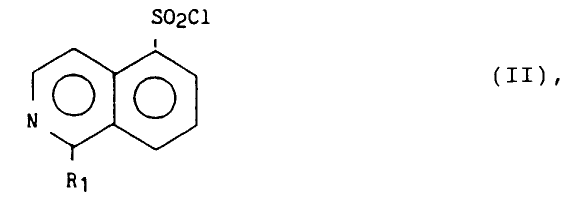 in which R₁ has the same meaning as in the general formula (I),
is reacted
- either with a compound of formula (III): 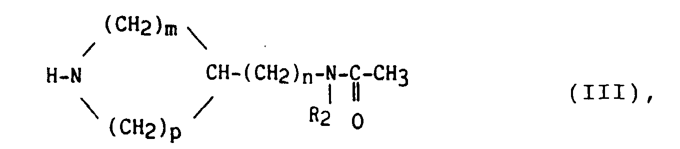 in which R₂, n, m and p have the same meaning as in the general formula (I),
to obtain, after acid hydrolysis, a compound of formula (IV): 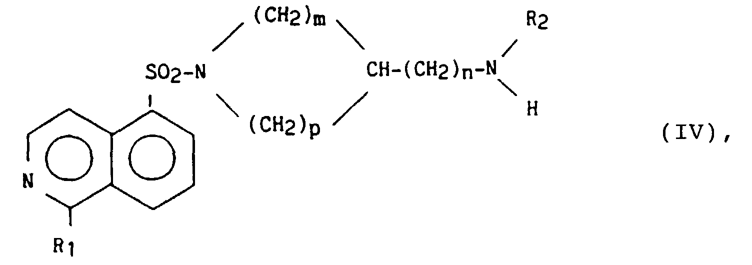 in which R₁, R₂, n, m and p are as defined above, which is reacted with a compound of formula (V): 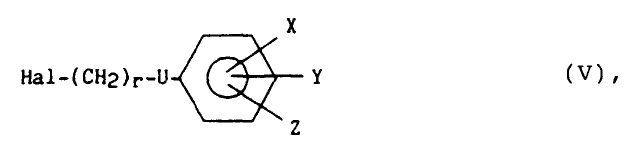 in which X, Y, Z, U and r have the same meaning as in the general formula (I) and Hal represents a halogen atom, to obtain a compound of formula (I): 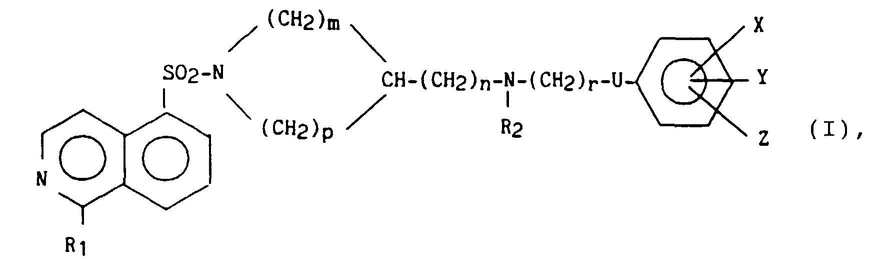 in which R₁, R₂, U, X, Y, Z, n, m, p and r are as defined in the general formula (I),
- or with a compound of formula (VI): 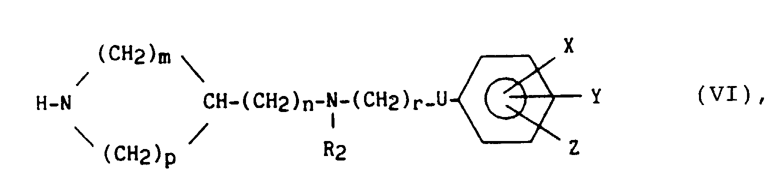 in which R₂, U, X, Y, Z, n, m, p and r are as defined above, to obtain a compound of formula (I), the isomers of which, where applicable, are separated, and which, if desired, is converted into a salt with a pharmaceutically acceptable acid,
which compound of formula (I) is purified, if desired, by a method of crystallisation and/or chromatography.

10. Pharmaceutical composition containing as active ingredient at least one compound according to claims 1 to 8 in combination with one or more inert, non-toxic, pharmaceutically acceptable excipients or vehicles.

11. Pharmaceutical composition according to claim 10 which can be used in the treatment and prevention of disorders due to or associated with phenomena of tissue damage, and in particular in the treatment and prevention of myocardial and peripheral ischaemia, of vascular disorders, of arterial hypertension, of cardiac insufficiency, of disturbances in cardiac rhythm, of disorders associated with arterial ageing and with atherosclerosis, of metabolic pathologies which constitute a cardiovascular risk factor, of thromboses, of disorders relating to the bronchial, digestive, urinary and uterine smooth muscles, of conditions of tissue damage associated with ageing, with ischaemia, with inflammation or with cell proliferation,
and which can also be used for the prevention of vascular re-stenoses or thromboses following bypass surgery, vascular, especially coronary, dilatation, or other forms of vascular repermeabilisation.

## Claims (Claims for the following Contracting State(s): ES, GR)

1. Process for the preparation of the compounds of the general formula (I): 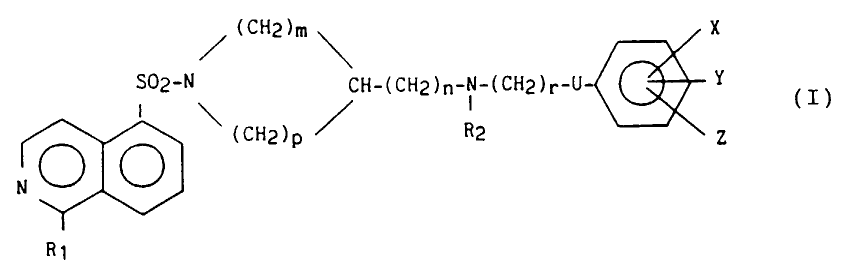 in which:
n is 0 or an integer from 1 to 3,
m and p are integers from 1 to 4 with the proviso that the sum of m+p is 2, 3, 4 or 5,
r is an integer from 1 to 6,
R₁ represents a hydrogen atom, a hydroxy group or a chlorine atom,
R₂ represents:
- a hydrogen atom,
- a formyl group,
- a group -A, -CO-A or -CO-O-A, wherein A represents a lower alkyl, lower alkenyl, lower alkynyl, cycloalkyl or cycloalkyl-lower alkyl group,
- a -(CH₂)_{q}-phenyl group or a substituted -(CH₂)_{q}-phenyl group, wherein q represents 0 or an integer from 1 to 4,
- a -CO-phenyl group or a substituted -CO-phenyl group,
- a -CO-O-phenyl group or a substituted -CO-O-phenyl group, or
- a group -CO-NR₄R₅, wherein R₄ and R₅
- which may be identical or different, each represent a hydrogen atom or a group selected from lower alkyl, lower alkenyl, lower alkynyl, phenyl and phenyl-lower alkyl, or
- together with the nitrogen atom carrying them, form a saturated ring having from 4 to 7 ring members,
X, Y and Z, which may be identical or different, each independently of the others represents a hydrogen atom, a halogen atom, or a group selected from lower alkyl, lower alkoxy, nitro, amino, cyano, acetamido and carboxamido, or X and Y, or X and Z, together form, with the two carbon atoms of the phenyl nucleus carrying them, a furan, dihydrofuran or benzene ring,
U represents an oxygen atom, a sulphur atom or a group selected from: carbonyl, sulphinyl, sulphonyl, -NH-CO-, -CO-NH-, -O-(CH₂)_{r'}-O- wherein r' represents an integer 2 or 3, -(CH₂)_{r"}-O-(CH₂)_{r'"}- wherein r" and r'" each represent integers 1 or 2, and 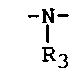 wherein R₃ represents:
- a hydrogen atom,
- a formyl group,
- a group -A, -CO-A or -CO-O-A, wherein A represents a lower alkyl, lower alkenyl, lower alkynyl, cycloalkyl or cycloalkyl-lower alkyl group,
- a -(CH₂)_{q}-phenyl group or a substituted -(CH₂)_{q}-phenyl group, wherein q represents 0 or an integer from 1 to 4,
- a -CO-phenyl group or a substituted -CO-phenyl group,
- a -CO-O-phenyl group or a substituted -CO-O-phenyl group, or
- a group -CO-NR₄R₅, wherein R₄ and R₅ are as defined above,
[wherein the term "substituted" relating to the groups -(CH₂)_{q}-phenyl, -CO-phenyl and -CO-O-phenyl means that those groups may be substituted by one or more radicals selected from: lower alkyl, lower alkoxy, hydroxy, halogen and trifluoromethyl,
- the terms "lower alkyl" and "lower alkoxy" indicate straight-chained or branched saturated carbon-containing groups containing from 1 to 6 carbon atoms,
- the terms "lower alkenyl" and "lower alkynyl" designate straight-chained or branched unsaturated groups containing from 2 to 6 carbon atoms, and
- the term "cycloalkyl" designates a saturated carbon-containing ring containing from 3 to 8 ring members,] the possible optical isomers thereof and, where applicable, the addition salts thereof with a pharmaceutically acceptable acid,
characterised in that:
- a compound of formula (II): 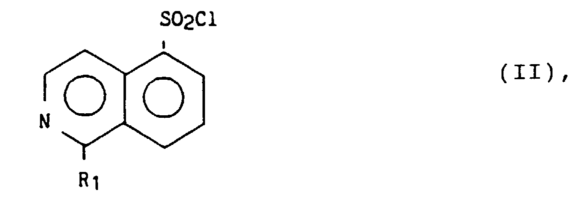 in which R₁ has the same meaning as in the general formula (I),
is reacted
a) either with a compound of formula (III): 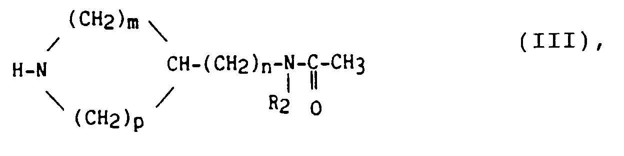 in which R₂, n, m and p have the same meaning as in the general formula (I),
to obtain, after acid hydrolysis, a compound of formula (IV): 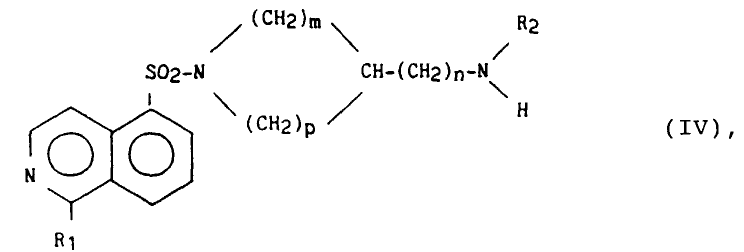 in which R₁, R₂, n, m and p are as defined above, which is reacted with a compound of formula (V): 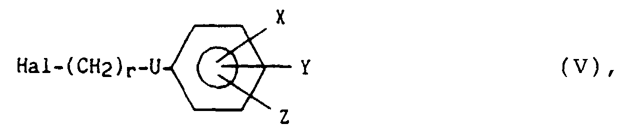 in which X, Y, Z, U and r have the same meaning as in the general formula (I) and Hal represents a halogen atom;
b) or with a compound of formula (VI): 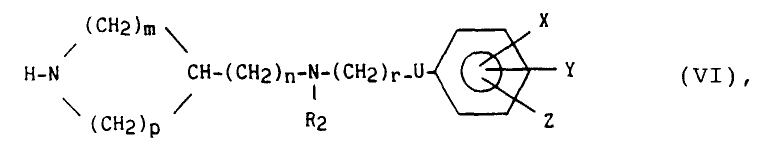 in which R₂, U, X, Y, Z, n, m, p and r are as defined above,
and, where applicable, the isomers of the resulting compound I are separated and/or, if desired, the resulting compound I is treated with a pharmaceutically acceptable acid to give the corresponding acid addition salts.
